(19) Europäisches Patentamt
European Patent Office
Office européen des brevets

(11) Veröffentlichungsnummer: **0 061 113**
**B1**

# (12) EUROPÄISCHE PATENTSCHRIFT

(45) Veröffentlichungstag der Patentschrift: **06.06.84**

(51) Int. Cl.³: **C 07 C 79/36**, C 07 C 76/02

(21) Anmeldenummer: **82102069.0**

(22) Anmeldetag: **15.03.82**

(54) **Verfahren zur Herstellung von gegebenenfalls substituierten Fluor-nitro-benzaldehyden.**

(30) Priorität: **24.03.81 DE 3111421**

(43) Veröffentlichungstag der Anmeldung: **29.09.82 Patentblatt 82/39**

(45) Bekanntmachung des Hinweises auf die Patenterteilung: **06.06.84 Patentblatt 84/23**

(84) Benannte Vertragsstaaten: **CH DE FR GB LI**

(56) Entgegenhaltungen:
**US - A - 3 064 058**

**Patent Abstracts of Japan Band 5, Nr. 71, 13. Mai 1981**

(73) Patentinhaber: **BAYER AG, Konzernverwaltung RP Patentabteilung, D-5090 Leverkusen 1 Bayerwerk (DE)**

(72) Erfinder: **Marzolph, Gerhard, Dr., Roggendorfstrasse 65, D-5000 Köln 80 (DE)**
Erfinder: **Blank, Heinz Ulrich, Dr., Am Geusfelde 35, D-5068 Odenthal (DE)**

EP 0 061 113 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von gegebenenfalls substituierten Fluor-nitro-benzaldehyden durch Umsetzung von gegebenenfalls substituierten Halogen-nitro-benzaldehyden mit einem Alkalimetallfluorid in einem inerten Lösungsmittel.

Bisher wurde zur Herstellung von Fluor-nitro-benzaldehyden stets von fluorhaltigen Benzolverbindungen ausgegangen, bevor die Aldehyd- oder Nitro-Gruppe oder beide eingeführt wurden.

So ist in J.Chem.Soc. 1961, Seite 5418 die Herstellung von 5-Fluor-2-nitro-benzaldehyd aus dem teuren 3-Fluortoluol beschrieben, wobei zunächst die Methylgruppe durch Chlorierung und Verseifung in die Aldehydgruppe umgewandelt wird und anschließend nitriert wird.

In Org. Synthesis, Col. Vol. V, Seite 825 ist die Herstellung von gegebenenfalls Fluor-substituierten o-Nitrobenzaldehyden beschrieben, wobei man vom o-Nitrotoluol ausgeht und über die Stufen des Benzylpyridiniumbromids und des p-Dimethylaminophenyl-$\alpha$-phenylnitrons und dessen Spaltung mit Schwefelsäure zum entsprechend substituierten Benzaldehyd gelangt.

Weiterhin ist aus Chem.Ber. 90, 1586 (1957) die Nitrierung von 4-Fluorbenzaldehyd zu 4-Fluor-3-nitro-benzaldehyd bekannt.

Aus US 3 064 058 ist die Umsetzung von p-Nitro-chlorbenzol mit Kaliumfluorid bei 240° C in Tetramethylensulfon zu p-Nitro-fluorbenzol bekannt.

Es ist jedoch auch bekannt, daß Kaliumfluorid in wasserfreien Medien als stark basischer Katalysator, beispielsweise für decarboxylierende Cyclisierungen und Kondensationsreaktionen unter Einschluß von Oxogruppen verwendet werden kann (Fieser, Reagents for Organic Synthesis, Seite 933, John Wiley and Sons Inc., 1967).

Es wurde nun überraschend gefunden, daß der Austausch eines von Fluor verschiedenen Halogenatoms an Halogennitro-benzaldehyden mit einem Alkalimetallfluorid durchgeführt werden kann, ohne daß Nebenreaktionen an und mit der Aldehydgruppe unter dem Einfluß des Alkalimetallfluorids eintreten. Darüber hinaus kann bei überraschend niedrigen Temperaturen gearbeitet werden.

Die Erfindung betrifft somit ein Verfahren zur Herstellung von gegebenenfalls substituierten Fluor-nitro-benzaldehyden der Formel

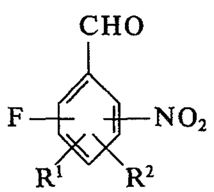

(I)

in der

$R^1$ und $R^2$ unabhängig voneinander für Wasserstoff, Alkyl, Aryl oder Aralkyl stehen und weiterhin für den Fall, daß Fluor und Nitro zueinander ortho- oder paraständig sind, ein zur Nitrogruppe ortho- oder paraständiges zweites Fluoratom oder ein zur Nitrogruppe metaständiges Chlor, Brom oder Jod bedeuten können, das dadurch gekennzeichnet ist, daß man Halogen-nitro-benzaldehyde der Formel

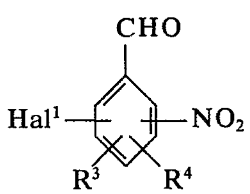

(II)

in der

$R^3$ und $R^4$ unabhängig voneinander für Wasserstoff, Alkyl, Aryl, Aralkyl, Chlor, Brom oder Jod und $Hal^1$ für Chlor, Brom oder Jod stehen,

mit einem Alkalimetallfluorid in Gegenwart eines polaren, aprotischen Lösungsmittels in weitgehend wasserfreiem Medium bei einer Temperatur von 50 bis 250° C umsetzt und nach beendeter Umsetzung die im Reaktionsgemisch vorliegenden Alkalimetallsalze mit Wasser aufnimmt und die Fluor-nitro-benzaldehyde durch Destillation oder Wasserdampfdestillation gewinnt.

Als Alkyl sei beispielsweise ein geradkettiger oder verzweigter aliphatischer Kohlenwasserstoffrest mit 1 bis 20, bevorzugt 1 bis 10, besonders bevorzugt 1 bis 4 C-Atomen genannt, wie Methyl, Ethyl, Propyl, Isopropyl, Butyl, Isobutyl, Hexyl, Isohexyl, Octyl, Isooctyl, Stearyl oder Eicosyl. In ganz besonders bevorzugter Weise sei Methyl oder Ethyl genannt.

Als Aryl sei beispielsweise Phenyl, Diphenyl, Naphthyl oder Anthryl, bevorzugt Phenyl, genannt. Als

Aralkyl seien beispielsweise Substituenten genannt, die als aromatischen Teil Phenyl, Diphenyl, Naphthyl oder Anthryl enthalten und deren aliphatischer Teil bis zu 4 C-Atomen enthält. Bevorzugtes Aralkyl ist Benzyl.

Als Beispiel für erfindungsgemäß einsetzbare Halogen-nitro-benzaldehyde seien genannt:

4-Chlor-3-nitrobenzaldehyd, 2-Chlor-5-nitrobenzaldehyd,
5-Chlor-2-nitrobenzaldehyd, 2,6-Dichlor-3-nitrobenzaldehyd,
3-Brom-4-chlor-5-nitrobenzaldehyd, 2,4-Di-chlor-5-nitrobenzaldehyd,
2-Chlor-4-methyl-5-nitrobenzaldehyd, 3,5-Dichlor-2-nitrobenzaldehyd,
3,6-Dichlor-2-nitrobenzaldehyd, 2,5-Dichlor-3-nitrobenzaldehyd,
3,4,5-Trichlor-2-nitrobenzaldehyd und 4-Brom-3,5-dichlor-2-nitrobenzaldehyd.

Solche Halogen-nitro-benzaldehyde sind bekannt und können beispielsweise durch Nitrierung aus den zugehörigen Halogen-benzaldehyden hergestellt werden (Friedländer, Fortschritte der Teerfarbenfabrikation, Band 3, Seite 63, Verlag Julius Springer, Berlin 1896).

In bevorzugter Weise werden Halogen-nitro-benzaldehyde der Formel

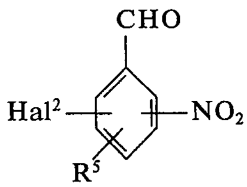

(III)

eingesetzt, in der

$R^5$ für Wasserstoff, $C_1$-$C_4$-Alkyl, Phenyl, Benzyl, Chlor oder Brom und
$Hal^2$ für Chlor oder Brom stehen.

In ganz besonders bevorzugter Weise werden Halogen-nitro-benzaldehyde der Formel

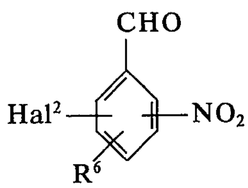

(IV)

eingesetzt, in der
$R^6$ für Wasserstoff, Methyl oder Ethyl steht und
$Hal^2$ Chlor oder Brom bedeutet, wobei $Hal^2$ in ortho- oder para-Stellung zur Nitrogruppe steht.

Als Alkalimetallfluorid für das erfindungsgemäße Verfahren sei beispielsweise Natriumfluorid, Kaliumfluorid, Rubidiumfluorid oder Cäsiumfluorid, bevorzugt Kaliumfluorid, genannt. Das Alkalimetallfluorid wird in einer Menge von 1 bis 10 Mol, bevorzugt 1,1 bis 3 Mol je Äquivalent austauschbares Halogenatom eingesetzt. Unterstöchiometrische Mengen Alkalimetallfluorid führen zu einer unvollständigen Umsetzung des Halogen-nitro-benzaldehyds. Eine solche Reaktionsweise ist zwar grundsätzlich möglich, jedoch wegen der Ausbeuteminderung nicht vorteilhaft. Es wurde gefunden, daß Halogen, das zur Nitrogruppe ortho- oder para-ständig ist, schneller gegen Fluor ausgetauscht werden kann als ein meta-ständiges Halogen. Für den Fall, daß der als Startmaterial einzusetzende Halogen-nitro-benzaldehyd ein zweites Halogenatom enthält, kann daher dieses zweite Halogenatom ebenfalls gegen Fluor ausgetauscht werden, wenn es ebenso wie das erste Halogenatom zur Nitrogruppe in ortho- oder para-Position steht. Steht hingegen nur das erste Halogenatom in ortho- oder para-Position zur Nitrogruppe und das zweite Halogenatom in der meta-Position zur Nitrogruppe, so wird im allgemeinen nur dieses erste Halogenatom gegen Fluor ausgetauscht.

Das einzusetzende Alkalimetallfluorid soll weitgehend trocken sein. Als weitgehend trocken sei hierbei ein solcher Wassergehalt genannt, der im Reaktionsgemisch einen Wassergehalt von höchstens 1 Gew.-%, beispielsweise 0,001 bis 1 Gew.-%, ermöglicht. Die Trocknung des Alkalimetallfluorids kann nach allgemein bekannten Methoden durchgeführt werden. In der Regel genügt schon eine Trocknung bei 170 bis 200° C im Trockenschrank während einer Zeit von 5 bis 10 Stunden.

Das erfindungsgemäße Verfahren wird in einem polaren aprotischen Lösungsmittel durchgeführt. Solche Lösungsmittel sind beispielsweise am Stickstoff vollständig substituierte Säureamide, wie Dimethylformamid, Dimethylacetamid, Diethylformamid, Diethylacetamid, N-Methylpyrrolidon oder Hexamethylphosphorsäuretriamid, ferner Sulfone, wie Dimethylsulfon, Tetramethylsulfon oder Diphenylsulfon, weiterhin Sulfoxide, wie Dimethylsulfoxid, Glykolether oder Polyglykolether, wie Dimethoxyethan, Diethoxyethan, Diglyme bzw. Triglyme oder Tetraglykoldimethylether, weiterhin Nitrile, wie Acetonitril oder Benzonitril, und weiterhin Nitrobenzol. Bevorzugt sind von den am Stickstoff vollständig substituierten Säureamiden die N,N-disubstituierten Carbonsäureamide, weiterhin bevorzugt sind die Sulfone. Die genannten Lösungsmittel können einzeln oder als Gemische verschiedener der genannten Substanzen eingesetzt werden. Die Menge des Lösungsmittels wird so bemessen, daß sie mit den Reaktionspartnern zusammen eine gut rührbare Suspension bilden. Beispielsweise seien 0,1 bis 20 Gew.-Teile Lösungsmittel oder Lösungsmittelgemisch pro 1 Gew.-Teil der Summe aus dem Halogen-nitro-benzaldehyd und dem Alkalimetallfluorid, bevorzugt 0,5 Gew.-Teile bis 5 Gew.-Teile, genannt. Die genannten Lösungsmittel sollen ebenso wie das Alkalimetallfluorid für das erfindungsgemäße Verfahren weitgehend wasserfrei sein, wobei für den Wassergehalt die oben angegebenen Grenzen gelten. Dieser Grad an Wasserfreiheit des Lösungsmittels kann durch an sich bekannte Methoden erreicht werden, wie Zusatz eines inerten Trockenmittels, Andestillieren der Lösungsmittelmenge, bis durch azeotropes Abdestillieren des Wassers mit einem Teil des Lösungsmittels der geforderte Wassergehalt erreicht wurde oder auch durch azeotrope Destillation des Wassers aus dem Lösungsmittel unter Verwendung eines Schleppmittels für das Wasser. Es ist weiterhin möglich, das Alkalimetallfluorid und das Lösungsmittel oder Lösungsmittelgemisch vorzulegen, bei denen das Salz oder das Lösungsmittel oder beide einen Wassergehalt oberhalb der genannten Grenze von 1 Gew.-% aufweisen, und sodann durch Andestillieren oder durch azeotrope Destillation unter Zusatz eines Schleppmittels den Wassergehalt dieser Salz-Lösungsmittel-Suspension auf das geforderte Maß zu bringen.

Das erfindungsgemäße Verfahren wird bei einer Temperatur von 50 bis 250° C, bevorzugt von 100 bis 180° C, durchgeführt. Zur Kontrolle der erfindungsgemäßen Reaktion kann es günstig sein, das verwendete Lösungsmittel unter Rückflußbedingungen sieden zu lassen. Hierzu kann in bekannter Weise außer bei atmosphärischem Druck auch bei erniedrigtem oder erhöhtem Druck gearbeitet werden. Die Einstellung von Rückflußbedingungen und gegebenenfalls die Einstellung eines vom Normaldruck abweichenden Druckes sind für den Erfolg des erfindungsgemäßen Verfahrens nicht kritisch.

Das erfindungsgemäße Verfahren kann sowohl kontinuierlich als auch diskontinuierlich durchgeführt werden.

Das Zusammengeben der Reaktionspartner und des Lösungsmittels in den Reaktor kann in beliebiger Reihenfolge durchgeführt werden. Vielfach wird das Lösungsmittel vorgelegt und die beiden Reaktionspartner eingetragen. Die Halogen-nitro-benzaldehyde werden hierbei als trockene Substanz in fester oder flüssiger Form, gegebenenfalls auch als Lösung in einem Teil des verwendeten Lösungsmittels in den Reaktor gegeben. Gegebenenfalls kann vor der Zugabe der Halogen-nitro-benzaldehyde in der oben beschriebenen Weise der Wassergehalt des Lösungsmittels, gegebenenfalls zusätzlich des Alkalimetallfluorids, auf den erforderlichen Wert eingestellt werden.

Zur Isolierung der gegebenenfalls substituierten Fluor-nitro-benzaldehyde kann beispielsweise das Lösungsmittel abdestilliert und der verbleibende Rückstand mit Wasser verrührt werden. Aus dem hierbei entstehenden zweiphasigen System wird die wäßrige Phase abgetrennt und die organische Phase, beispielsweise durch Destillation, Extraktion oder Kristallisation, weiter zu den gereinigten Fluor-nitro-benzaldehyden aufgearbeitet. Das abdestillierte Lösungsmittel wird weitgehend zurückgewonnen und kann gegebenenfalls nach einer Reinigung erneut verwendet werden.

In einer anderen Aufarbeitungsvariante kann aber auch der gesamte Reaktionsansatz mit Wasser verrührt werden und der gegebenenfalls substituierte Fluor-nitro-benzaldehyd aus diesem Gemisch, beispielsweise durch Filtration, Extraktion oder auch Wasserdampfdestillation, abgetrennt werden.

Das erfindungsgemäße Verfahren bietet außer der eingangs beschriebenen überraschend unkomplizierten Durchführbarkeit eine Reihe technischer Vorteile:

1. Das Fluoratom wird erst am Ende der Reaktionssequenz eingeführt. Dadurch ist es möglich, mit großtechnisch anfallenden, preiswerten Ausgangssubstanzen zu arbeiten, beispielsweise mit Chlortoluol oder Chlor-benzaldehyd, die in bekannter Weise in die entsprechend substituierten Chlor-nitro-benzaldehyde umgewandelt werden.
2. Gegenüber anderen Verfahren zur Einführung des Fluoratoms durch Halogen-Fluor-Austausch kann bei vorteilhaft niedrigen Temperaturen gearbeitet werden.
3. Die bei vielen Verfahren des Standes der Technik zur Einführung von Fluor in eine aromatische Verbindung verwendeten schwierigen oder teuren Reagentien, wie Fluor, Fluorwasserstoff, Bortrifluorid oder Tetrafluoroborwasserstoff können zugunsten der unkompliziert handhabbaren Alkalimetallfluoride aufgegeben werden.

Die erfindungsgemäß herstellbaren Fluor-nitro-benzaldehyde sind wertvolle Ausgangssubstanzen zur Herstellung pharmazeutisch wirksamer Substanzen, beispielsweise zur Herstellung von verschmolzenen Pyrimidinen, die in der Behandlung von Allergien, wie Asthma, eingesetzt werden (DE-OS 2 418 498; US-PS 4 044 134). Weiterhin können sie zur Synthese von Benzyliden-hydrazino-(1,2,4)-triazolen eingesetzt werden, die zur Behandlung von Bluthochdruck geeignet sind (südafrikanische Patentanmeldung 7 703 470; zitiert nach C.A. 89, P 146 910 f).

Beispiel 1

4-Fluor-3-nitrobenzaldehyd

93 g (0,5 Mol) 4-Chlor-3-nitrobenzaldehyd und 58 g (1 Mol) Kaliumfluorid werden in 250 ml Dimethylformamid suspendiert. Die Reaktionsmischung enthält 0,02% $H_2O$. Man rührt eine Stunde bei 160°C und dampft das DMF im Vakuum ab. Der Rückstand wird gekühlt und mit 200 ml Wasser verrührt, mit Methylenchlorid extrahiert und die organische Phase getrocknet. Nach Abdampfen des Methylenchlorids erhält man 83 g 4-Fluor-3-nitrobenzaldehyd mit einem Gehalt von 90,2%.

Ausbeute: 88% der theoretischen Ausbeute.
Nach Umkristallisieren schmilzt der 4-Fluor-3-nitrobenzaldehyd bei 42 bis 44° C.

Beispiel 2

2-Fluor-5-nitrobenzaldehyd

55,7 g (0,3 Mol) 2-Chlor-5-nitrobenzaldehyd und 34,8 g (0,6 Mol) Kaliumfluorid werden in 250 ml DMF suspendiert und 0,5 Stunden bei 160°C gerührt. Der Ansatz wird anschließend auf 600 ml Wasser gegeben und der abgeschiedene Aldehyd mit Methylenchlorid extrahiert. Der Extrakt wird abgetrennt, getrocknet und eingedampft. Man erhält 48,9 g 2-Fluor-5-nitrobenzaldehyd mit einem Gehalt von 94,8%.

Ausbeute: 91% der theoretischen Ausbeute.
Nach Umkristallisieren schmilzt der 2-Fluor-5-nitrobenzaldehyd bei 53 bis 54° C.

Beispiel 3

5-Fluor-2-nitrobenzaldehyd

37 g (0,2 Mol) 5-Chlor-2-nitrobenzaldehyd und 23,2 g (0,4 Mol) Kaliumfluorid werden in 300 ml Dimethylacetamid bei 150° C 3 Stunden gerührt. Anschließend wird das Lösungsmittel im Vakuum abgezogen und der Rückstand mit 200 ml Wasser verrührt. Der Aldehyd wird mit Methylenchlorid extrahiert, die organische Phase abgetrennt, getrocknet und eingedampft. Man erhält 24 g 5-Fluor-2-nitrobenzaldehyd mit einem Gehalt von 86%.

Ausbeute: 60,7% der theoretischen Ausbeute.
Der 5-Fluor-2-nitrobenzaldehyd schmilzt nach Umkristallisieren bei 92 bis 94° C.

Beispiel 4

18,6 g (0, 1 Mol) 4-Chlor-3-nitrobenzaldehyd wird mit 6,4 g (0,11 Mol) Kaliumfluorid in 50 ml DMF bei 160° C gerührt. Nach 3 Stunden ist in der Reaktionsmischung nach Gaschromatographie kein 4-Chlor-3-nitrobenzaldehyd mehr nachweisbar. Dafür hat sich quantitativ der 4-Fluor-3-nitrobenzaldehyd gebildet.

Beispiel 5

18,6 g (0,1 Mol) 4-Chlor-3-nitrobenzaldehyd wird mit 8,7 g (0,15 Mol) Kaliumfluorid in 100 ml Sulfolan für 1 Stunde bei 180°C gerührt. Der Ansatz wird mit 300 ml Wasser verrührt und der Aldehyd mit Methylenchlorid extrahiert. Nach der üblichen Aufarbeitung erhält man 17,1 g 92%igen 4-Fluor-3-nitrobenzaldehyd.

Ausbeute: 92,5% der theoretischen Ausbeute.

Beispiel 6

18,6 g (0,1 Mol) 2-Chlor-5-nitrobenzaldehyd und 11,6 g (0,2 Mol) trockenes Kaliumfluorid werden in 50 ml Dimethylformamid 12 Stunden bei 100° C gerührt. Anschließend wird im Vakuum das Lösungsmittel abdestilliert und der Rückstand in 100 ml Wasser verrührt. Der rohe 2-Fluor-5-nitrobenzaldehyd wird abgesaugt, mit Wasser gewaschen und getrocknet. Man erhält 16,7 g 2-Fluor-5-nitrobenzaldehyd mit einem Gehalt von 96%.

Ausbeute: 94,8% der theoretischen Ausbeute.

**Patentansprüche**

1. Verfahren zur Herstellung von gegebenenfalls substituierten Fluor-nitro-benzaldehyden der Formel

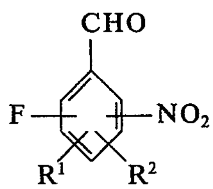

in der

$R^1$ und $R^2$ unabhängig voneinander für Wasserstoff, Alkyl, Aryl, oder Aralkyl stehen und weiterhin für den Fall, daß Fluor und Nitro zueinander ortho- oder para-ständig sind, ein zur Nitrogruppe ortho- oder para-ständiges zweites Fluoratom oder ein zur Nitrogruppe meta-ständiges Chlor, Brom oder Jod bedeuten können,

dadurch gekennzeichnet, daß man Halogen-nitro-benzaldehyde der Formel

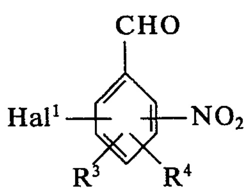

in der

$R^3$ und $R^4$ unabhängig voneinander für Wasserstoff, Alkyl, Aryl, Aralkyl, Chlor, Brom oder Jod und $Hal^1$ für Chlor, Brom oder Jod stehen,

mit einem Alkalimetallfluorid in Gegenwart eines polaren, aprotischen Lösungsmittels in weitgehend wasserfreiem Medium bei einer Temperatur von 50 bis 250° C umsetzt und nach beendeter Umsetzung die im Reaktionsgemisch vorliegenden Alkalimetallsalze mit Wasser aufnimmt und die Fluor-nitrobenzaldehyde durch Destillation oder Wasserdampfdestillation gewinnt.

2. Verfahren nach Anspruch 1, dadurch gekennzeichnet, daß man Halogen-nitro-benzaldehyde der Formel

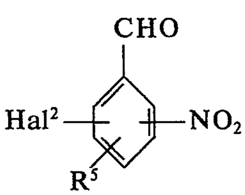

einsetzt, in der

$R^5$ für Wasserstoff, $C_1-C_4$-Alkyl, Phenyl, Benzyl, Chlor oaer Brom und
$Hal^2$ für Chlor oder Brom steht.

3. Verfahren nach Anspruch 1, dadurch gekennzeichnet, daß man Halogen-nitro-benzaldehyde der Formel

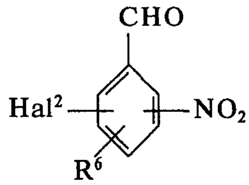

einsetzt, in der

$R^6$ für Wasserstoff, Methyl oder Ethyl steht und
$Hal^2$ Chlor oder Brom bedeutet, wobei $Hal^2$ in ortho- oder para-Stellung zur Nitrogruppe steht.

4. Verfahren nach Ansprüchen 1 bis 3, dadurch gekennzeichnet, daß als Alkalimetallfluorid Kaliumfluorid eingesetzt wird.

5. Verfahren nach Anspruch 1, dadurch gekennzeichnet, daß 1 bis 10 Mol Alkalimetallfluorid pro gegen Fluor auszutauschendes Äquivalent Halogen eingesetzt werden.

6. Verfahren nach Ansprüchen 1 und 5, dadurch gekennzeichnet, daß 1,1 bis 3 Mol Alkalimetallfluorid pro gegen Fluor auszutauschendes Äquivalent Halogen eingesetzt werden.

7. Verfahren nach Anspruch 1, dadurch gekennzeichnet, daß als Lösungsmittel ein am Stickstoff vollständig substituiertes Säureamid, ein Sulfon, ein Sulfoxid, ein Glykolether, ein Polyglykolether, ein Nitril oder Nitrobenzol oder ein Gemisch aus solchen Lösungsmitteln eingesetzt wird.

8. Verfahren nach Ansprüchen 1 und 7, dadurch gekennzeichnet, daß als Lösungsmittel ein N,N-disubstituiertes Carbonsäureamid oder ein Sulfon oder ein Gemisch hieraus eingesetzt wird.

9. Verfahren nach Anspruch 1, 7 und 8, dadurch gekennzeichnet, daß der Wassergehalt des Reaktionsgemisches höchstens 1 Gew.-% beträgt.

10. Verfahren nach Anspruch 1, dadurch gekennzeichnet, daß man bei einer Temperatur von 100 bis 180° C arbeitet.

## Claims

1. Process for the preparation of optionally substituted fluoro-nitro-benzaldehydes of the formula

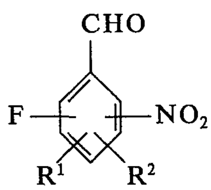

in which

$R^1$ and $R^2$ independently of one another represent hydrogen, alkyl, aryl or aralkyl, and furthermore, in the case in which fluorine and nitro are located ortho or para to one another, can denote a second fluorine atom located ortho or para to the nitro group, or a chlorine, bromine or iodine located meta to the nitro group,

characterized in that halogeno-nitro-benzaldehydes of the formula

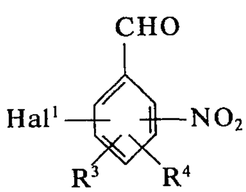

in which

$R^3$ and $R^4$ independently of one another represent hydrogen, alkyl, aryl, aralkyl, chlorine, bromine or iodine, and
$Hal^1$ represents chlorine, bromine or iodine,

are reacted with an alkali metal fluoride in the presence of a polar aprotic solvent, in a substantially anhydrous medium at a temperature of from 50 to 250° C, and, after the reaction is complete, the alkali metal salts present in the reaction mixture are taken up with water, and the fluoro-nitro-benzaldehydes are obtained by distillation or steam distillation.

2. Process according to claim 1, characterized in that halogeno-nitro-benzaldehydes of the formula

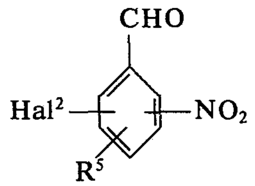

in which

$R^5$ represents hydrogen, $C_1-C_4$-alkyl, phenyl, benzyl, chlorine or bromine, and
$Hal^2$ represents chlorine or bromine,

are employed.

3. Process according to claim 1, characterized in that halogeno-nitro-benzaldehydes of the formula

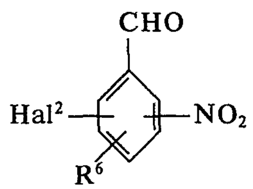

in which

$R^6$ represents hydrogen, methyl or ethyl, and
$Hal^2$ denotes chlorine or bromine, $Hal^2$ being located in the ortho or para position to the nitro group,

are employed.

4. Process according to claims 1 to 3, characterized in that potassium fluoride is employed as the alkali metal fluoride.

5. Process according to claim 1, characterized in that 1 to 10 mol of alkali metal fluoride are employed per halogen equivalent to be replaced by fluorine.

6. Process according to claims 1 and 5, characterized in that 1,1 to 3 mol of alkali metal fluoride are employed per halogen equivalent to be replaced by fluorine.

7. Process according to claim 1, characterized in that an acid amide which is completely substituted at the nitrogen, a sulphone, a sulphoxide, a glycol ether, a polyglycol ether, a nitrile or nitrobenzene, or a mixture of such solvents, is employed as the solvent.

8. Process according to claims 1 and 7, characterized in that an N,N-disubstituted carboxamide or a sulphone, or a mixture of these, is employed as the solvent.

9. Process according to claims 1, 7 and 8, characterized in that the water content of the reaction mixture is at most 1% by weight.

10. Process according to claim 1, characterized in that the reaction is carried out at a temperature of from 100 to 180° C.

## Revendications

1. Procédé de production de fluoronitrobenzaldéhydes éventuellement substitués de formule

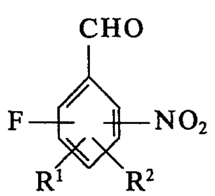

dans laquelle

$R^1$ et $R^2$ représentent, indépendamment, l'un de l'autre, l'hydrogène, un groupe alkyle, aryle ou aralkyle et, en outre, au cas où le fluor et le groupe nitro sont en position ortho ou para l'un par rapport à l'autre, ils peuvent représenter un second atome de fluor en position ortho ou para par rapport au groupe nitro ou un atome de chlore, de brome ou d'iode en position méta par rapport au groupe nitro,

caractérisé en ce qu'on fait réagir des halogénonitrobenzaldéhydes de formule

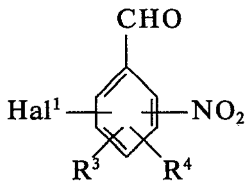

dans laquelle

$R^3$ et $R^4$ représentent, indépendamment l'un de l'autre, l'hydrogène, un groupe alkyle, aryle, aralkyle, du chlore, du brome ou de l'iode et
$Hal^1$ désigne du chlore, du brome ou de l'iode,

avec un fluorure de métal alcalin en présence d'un solvant aprotique polaire en milieu principalement anhydre à une température de 50 à 250° C et une fois la réaction terminée, on reprend par l'eau les sels de métaux alcalins présents dans le mélange réactionnel et on obtient les fluoronitrobenzaldéhydes par distillation ou par distillation à la vapeur d'eau.

2. Procédé suivant la revendication 1, caractérisé en ce qu'on utilise des halogénonitrobenzaldéhydes de formule

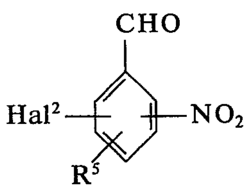

dans laquelle

$R^5$ représente l'hydrogène, un groupe alkyle en $C_1$ à $C_4$, phényle, benzyle, du chlore ou du brome et
$Hal^2$ représente du chlore ou du brome.

3. Procédé suivant la revendication 1, caractérisé en ce qu'on utilise des halogénonitrobenzaldéhydes de formule

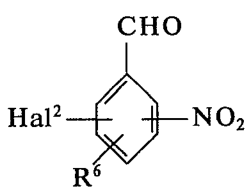

dans laquelle

$R^6$ représente de l'hydrogène, un groupe méthyle ou éthyle et
$Hal^2$ désigne du chlore ou du brome, $Hal^2$ étant en position ortho ou para par rapport au groupe nitro.

4. Procédé suivant les revendications 1 à 3, caractérisé en ce qu'on utilise le fluorure de potassium comme fluorure de métal alcalin.

5. Procédé suivant la revendication 1, caractérisé en ce qu'on utilise 1 à 10 moles de fluorure de métal alcalin par équivalent d'halogène à échanger contre du fluor.

6. Procédé suivant les revendications 1 à 5, caractérisé en ce qu'on utilise 1,1 à 3 moles de fluorure de métal alcalin par équivalent d'halogène à échanger contre du fluor.

7. Procédé suivant la revendication 1, caractérisé en ce qu'on utilise comme solvant un amide d'acide entièrement substitué sur l'azote, une sulfone, un sulfoxyde, un éther de glycol, un éther de polyglycol, un nitrile ou le nitrobenzène ou un mélange de ces solvants.

8. Procédé suivant les revendications 1 et 7, caractérisé en ce qu'on utilise comme solvant un amide

d'acide carboxylique N,N-disubstitué ou une sulfone ou un mélange de ces solvants.

9. Procédé suivant les revendications 1, 7 et 8, caractérisé en ce que la teneur en eau du mélange réactionnel s'élève au mamimum à 1% en poids.

10. Procédé suivant la revendication 1, caractérisé en ce qu'on opère à une température de 100 à 180° C.